# FASCICULE DE BREVET EUROPEEN

(11) **EP 0 634 641 B1**
(45) Date de publication et mention de la délivrance du brevet: **28.01.1998**
(21) Numéro de dépôt: 94401471.1
(22) Date de dépôt: 28.06.1994
(51) Int. Cl.: G01N 15/04, G01N 33/18

(54) **Dispositif pour mesurer en continu la concentration des matières en suspension d'un centrat**
Vorrichtung zur durchlaufenden Messung des Inhalts an suspendierten Materien in einem Zentrifugenablauf
Arrangement for continuous measurement of the concentration of suspended matter in a centrifugate

(30) Priorité: 15.07.1993 FR 9308707
(43) Date de publication de la demande: 18.01.1995
(73) Titulaire: DEGREMONT, 92508 Rueil-Malmaison Cédex (FR)
(72) Inventeur: Descamps, Patrick, F-78160 Marly Le Roi (FR)
(74) Mandataire: Armengaud Ainé, Alain

(56) Documents cités:
- WO-A-91/14171
- FR-A- 2 299 636
- KORRESPONDENZ ABWASSER, vol.37, no.4, 1 Avril 1990 pages 386 - 392 WINTER 'OPTIMIERTE FLOCKUNGSMITTELDOSIERUNG,ETC.'
- PATENT ABSTRACTS OF JAPAN vol. 8, no. 286 (P-324) (1723) 27 Décembre 1984 & JP-A-59 148 850 (HITACHI) 25 Août 1984
- PATENT ABSTRACTS OF JAPAN vol. 8, no. 159 (P-289) (1596) 24 Juillet 1984 & JP-A-59 057 161 (SHIMAZU) 2 Avril 1984
- PATENT ABSTRACTS OF JAPAN vol. 7, no. 206 (P-222) (1351) 10 Septembre 1983 & JP-A-58 102 153 (FUJI DENKI) 17 Juin 1983
- PATENT ABSTRACTS OF JAPAN vol. 11, no. 2 (P-532) (2449) 6 Janvier 1987 & JP-A-61 181 939 (MITSUBISHI) 14 Août 1986

## Description

La présente invention est relative à un dispositif permettant de mesurer en continu, d'une façon fiable la concentration en matières en suspension (MeS). ou de turbidité, d'un centrat provenant d'une centrifugeuse ou d'une machine à déshydratation. L'invention s'applique plus généralement à la mesure de la concentration en matières en suspension d'un effluent provenant d'une installation de traitement des eaux.

On sait qu'un centrat prélevé en sortie d'une centrifugeuse et sur lequel on doit effectuer une mesure de concentration, est très émulsionné et saturé en micro-bulles d'air. Quelque soit le capteur utilisé pour réaliser une mesure de (MeS) ou de turbidité, il faut éliminer les micro-bulles et les mousses contenues dans le fluide à mesurer pour que la mesure de concentration soit réalisée dans de bonnes conditions.

On connaît divers procédés et systèmes permettant de réaliser uniquement l'élimination des mousses ou des écumes lors des mesures de concentration.
JP-59-148 850 décrit un dispositif mécanique d'élimination des mousses en vue d'une mesure de la concentration de liquides chimiques.;
JP-59-057 161 décrit l'élimination des mousses à l'aide d'un disque rotatif comportant en outre une injection d'air au sein de la masse liquide devant subir la mesure de turbidité ;
JP-58-102 153 décrit un analyseur de turbidité, en particulier un équipement de dillution associé à des moyens d'élimination de la mousse, réalisés sous la forme d'un système de trop-plein ;
FR-A-2 299 636 se réfère à un procédé destiné à l'élimination des particules non colloïdales d'un liquide à clarifier. Cette publication antérieure prévoit une mesure continue de la turbidité colloïdale.

L'objet de la présente invention est de réaliser la mesure de MeS, en éliminant les micro-bulles, sans perdre de matières en suspension sur l'échantillon prélevé, de plus cette élimination ne doit pas entraîner une flottation des MeS contenus dans l'échantillon afin d'éviter que la mesure soit faussée.

En conséquence, cette invention concerne un procédé de mesure en continu de la concentration en matières en suspension ou de la turbidité d'un centrat provenant d'une centrifugeuse ou d'une machine de déshydratation selon lequel un échantillon du centrat prélevé par pompage en continu est délivré à une enceinte de désaération caractérisé en ce que :
ledit échantillon du centrat est amené tangentiellement dans la partie supérieure de ladite enceinte,
la vitesse ascensionnelle des bulles de gaz séparées du centrat dans ladite enceinte est au moins deux fois supérieure à la vitesse du liquide admis dans l'enceinte et ,
la mesure de la concentration est effectuée sur un centrat totalement désaéré dont la composition est identique à celle de l'échantillon prélevé.

Selon une autre caractéristique de l'invention, la vitesse de traversée de l'échantillon du centrat dans l'enceinte de désaération est de l'ordre de 0,1 à 0,5 cm/sec. et la vitesse ascensionnelle des bulles de gaz séparée du centrat dans l'enceinte de désaération est de l'ordre de 0,6 à 5 cm/sec.

La vitesse descendante de l'eau doit être plusieurs fois inférieure à celle des bulles de gaz.

L'invention vise également un dispositif pour la mise en oeuvre du procédé tel que spécifié ci-dessus caractérisé en ce qu'il comporte : une enceinte de désaération , de forme cylindro-conique dans la partie supérieure de laquelle est délivré l'échantillon du centrat, la zone inférieure conique de ladite enceinte recevant l'échantillon désaéré et,
- un capteur de concentration (16) recevant le centrat désaéré, à partir de ladite zone inférieure conique de ladite enceinte .

D'autres caractéristiques et avantages de la présente invention ressortiront de la description faite ci-après en référence au dessin annexé qui en illustre un exemple de réalisation. Sur le dessin :
- la figure 1 est une vue schématique représentant le dispositif objet de la présente invention et,
- la figure 2 représente l'implantation de ce dispositif à la sortie d'une centrifugeuse.

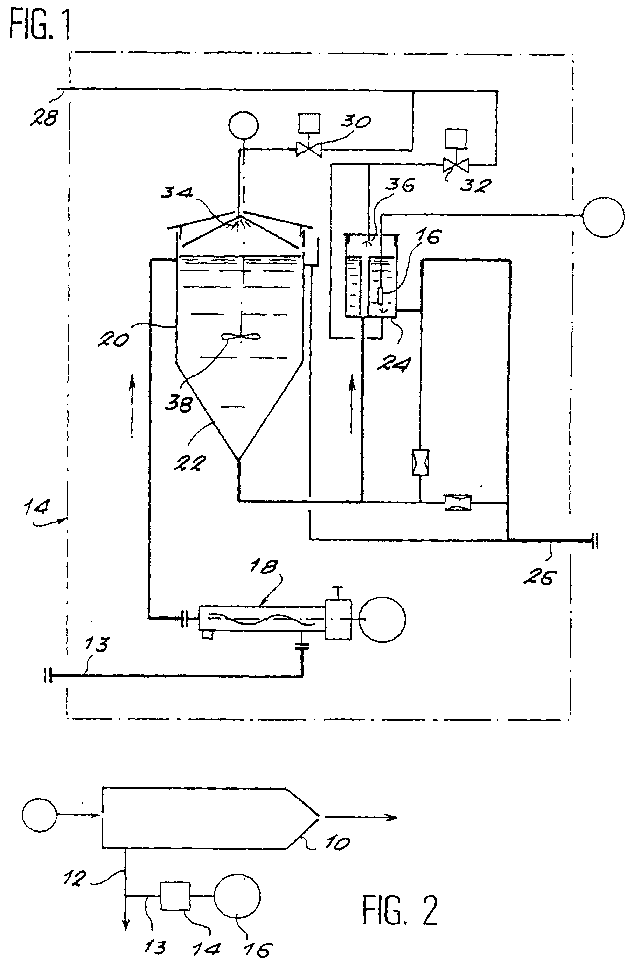

Ainsi qu'on l'a spécifié ci-dessus, le dispositif objet de la présente invention se propose de mesurer en continu, de façon fiable, un échantillon d'un centrat prélevé en continu à la sortie d'une centrifugeuse. La figure 2 représente de façon schématique une centrifugeuse 10 sur laquelle est prélevé en continu le centrat sur une canalisation de sortie 12, l'échantillon de centrat ainsi prélevé étant transmis au dispositif 14 selon l'invention de manière à en mesurer la concentration à l'aide d'un capteur de concentration classique 16. Ce capteur étant bien connu, il ne fera pas l'objet d'une description.

On se réfère maintenant à la figure 1 qui représente le dispositif selon l'invention.

Ce dispositif 14 comporte une pompe 18 délivrant le centrat prélevé à partir de la conduite 13 à une enceinte de désaération 20. Cette enceinte est conçue de façon à éliminer les micro-bulles contenues dans l'échantillon de centrat prélevé, afin de réaliser la mesure de concentration dans de bonnes conditions.

Cette enceinte de désaération 20 se présente sous la forme d'une cuve cylindro-conique dans la partie supérieure de laquelle l'échantillon de centrat arrive tangentiellement. L'intégralité de l'échantillon ainsi prélevé traverse l'enceinte de désaération 20 dans laquelle cette désaération est réalisée par séparation naturelle des micro-bulles contenues dans le centrat.

Selon l'invention, la vitesse de traversée de l'échantillon de centrat dans la cuve de désaération 20 est de l'ordre de 0,1 à 0,5 cm/sec. et la vitesse ascensionnelle des micro-bulles de gaz séparées de l'échantillon est de l'ordre de 0,6 à 5 cm/sec. Ainsi, la vitesse ascensionnelle de ces bulles de gaz est au moins deux fois supérieure à la vitesse du centrat dans l'enceinte de désaération.

L'enceinte de désaération 20 peut être munie d'un agitateur lent 38 permettant de mélanger dans la masse de l'échantillon du centrat les mousses susceptibles de s'accumuler à la surface de cette enceinte 20, permettant ainsi de mesurer la totalité des matières en suspension prélevées dans l'échantillon.

Cet agitateur mécanique peut être remplacé par des moyens empêchant la formation des mousses, ces moyens pouvant être réalisés sous la forme d'un dispositif de diffusion par aspersion de fines gouttelettes d'eau, placé au-dessus de la cuve 20 et fonctionnant selon un cycle déterminé pour empêcher la formation et/ou l'accumulation des mousses à la surface de ladite enceinte.

L'échantillon de centrat est désaéré lorsqu'il arrive dans la zone inférieure conique 22 de l'enceinte 20. Le capteur pourrait être positionné à cet emplacement. Toutefois, dans l'exemple de réalisation non limitatif, illustré sur la figure 1, le capteur 16 est placé dans une seconde enceinte 24, ceci pour des raisons pratiques d'exploitation.

Ainsi, ce capteur 16 effectue la mesure de la concentration sur un centrat totalement désaéré et contenant la même quantité de matières en suspension, comme à l'instant du prélèvement. L'échantillon de centrat sur lequel a été effectué la mesure de concentration en continu est éliminé vers l'égoût par une canalisation 26.

Afin que le dispositif selon l'invention soit toujours opérationnel dans les conditions optimales, il est pourvu d'un système de nettoyage périodique. Ce système a pour but de réaliser une vidange complète et rapide des deux enceintes 20 et 24, suivie d'un nettoyage de ces enceintes ainsi que du capteur 16.

Dans l'exemple de réalisation illustré par la figure 1, ce système de nettoyage et de rinçage comprend une alimentation 28 en eau sous pression, celle-ci étant délivrée respectivement par l'intermédiaire de vannes 30 et 32 à des buses respectivement 34 et 36 disposées à la partie supérieure de chacune des enceintes 20 et 24. Après la vidange complète de ces deux enceintes 20 et 24 par l'intermédiaire de la canalisation 26, l'eau sous pression diffusée par les buses de rinçage 34 et 36 assure le rinçage des enceintes 20 et 24 et du capteur 16. Pendant cette phase de nettoyage et de rinçage, le signal du capteur est mis en mémoire.
La mesure de la concentration est reprise automatiquement après remplissage des enceintes 20 et 24, le signal du capteur étant maintenu en mémoire également durant cette phase de remplissage.

Il demeure bien entendu que la présente invention n'est pas limitée aux exemples de réalisation décrits et/ou représentés ici mais qu'elle en englobe toutes les variantes.

## Revendications

1. Procédé de mesure en continu de la concentration en matières en suspension ou de la turbidité d'un centrat provenant d'une centrifugeuse ou d'une machine de déshydratation selon lequel un échantillon du centrat prélevé par pompage en continu est délivré à une enceinte de désaération caractérisé en ce que :
ledit échantillon du centrat est amené tangentiellement dans la partie supérieure de ladite enceinte (20),
la vitesse ascensionnelle des bulles de gaz séparées du centrat dans ladite enceinte est au moins deux fois supérieure à la vitesse du liquide admis dans l'enceinte et,
la mesure de la concentration est effectuée sur un centrat totalement désaéré dont la composition est identique à celle de l'échantillon prélevé.

2. Procédé selon la revendication 1 caractérisé en ce que la vitesse de traversée de l'échantillon du centrat dans l'enceinte de désaération (20) est de l'ordre de 0,1 à 0,5 cm/sec.

3. Procédé selon l'une des revendications 1 ou 2 caractérisé en ce que la vitesse ascensionnelle des bulles de gaz séparées du centrat dans l'enceinte de désaération (20) est de l'ordre de 0,6 à 5 cm/sec.

4. Dispositif pour la mise en oeuvre du procédé selon l'une quelconque des revendications 1 à 3 caractérisé en ce qu'il comporte : une enceinte de désaération (20), de forme cylindro-conique dans la partie supérieure de laquelle est délivré l'échantillon du centrat, la zone inférieure conique (22) de ladite enceinte recevant l'échantillon désaéré et,
- un capteur de concentration (16) recevant le centrat désaéré, à partir de ladite zone inférieure conique (22) de ladite enceinte (20).

5. Dispositif selon la revendication 4 caractérisé en ce que ladite enceinte de désaération (20) est munie de moyens d'élimination des mousses réalisés sous la forme d'un agitateur lent (38).

6. Dispositif selon l'une des revendications 4 ou 5 caractérisé en ce que le capteur de concentration (16), de type connu, est placé dans une seconde enceinte (24) qui reçoit le centrat à sa partie inférieure, en provenance de l'enceinte de désaération (20).

7. Dispositif selon l'une quelconque des revendications 4 à 6 caractérisé en ce qu'il comporte un système de nettoyage et de rinçage rapide, et fonctionnant selon un cycle déterminé afin d'éliminer la formation et l'accumulation éventuelle des mousses à la surface de ladite enceinte.

8. Dispositif selon la revendication 7 caractérisé en ce que ledit système de nettoyage rapide, périodique comporte des moyens pour assurer une vidange complète et rapide de l'enceinte de désaération (20) et de la seconde enceinte (24) contenant le capteur de concentration, et des moyens (34, 36) pour admettre de l'eau sous pression dans lesdites enceintes afin d'en assurer le rinçage, le signal du capteur étant mémorisé durant cette phase de nettoyage et de rinçage ainsi que pendant la phase de remplissage consécutive à cette phase de nettoyage.

## Patentansprüche

1. Verfahren zur kontinuierlichen Messung der Konzentration an suspendierten Stoffen oder der Trübung eines Zentrifugenablaufs, der aus einer Zentrifuge oder einem Entwässerungsapparat stammt, gemäß welchem eine durch ununterbrochenes Pumpen entnommene Probe des Zentrifugenablaufs einem Entlüftungsbehälter zugeleitet wird, **dadurch gekennzeichnet, daß**
- die Probe des Zentrifugenablaufs seitlich in den oberen Teil dieses Behälters (20) eingeleitet wird,
- die Aufstiegsgeschwindigkeit der vom Zentrifugenablauf getrennten Gasblasen in dem Behälter mindestens zweimal größer als die Geschwindigkeit der dem Behälter zugeführten Flüssigkeit ist und
- die Konzentrationsmessung an einem vollständig entlüfteten Zentrifugenablauf durchgeführt wird, dessen Zusammensetzung gleich der der entnommenen Probe ist.

2. Verfahren nach Anspruch 1, **dadurch gekennzeichnet, daß** die Strömungsgeschwindigkeit der Probe des Zentrifugenablaufs im Entlüftungsbehälter (20) etwa 0,1 bis 0,5 cm/s beträgt.

3. Verfahren nach Anspruch 1 oder 2, **dadurch gekennzeichnet, daß** die Aufstiegsgeschwindigkeit der vom Zentrifugenablauf getrennten Gasblasen im Entlüftungsbehälter (20) etwa 0,6 bis 5 cm/s beträgt.

4. Vorrichtung zur Durchführung des Verfahrens nach einem der Ansprüche 1 bis 3, **dadurch gekennzeichnet, daß** sie einen unten spitz zulaufenden zylindrischen Entlüftungsbehälter (20), in dessen oberen Teil die Probe des Zentrifugenablaufs eingeleitet wird, wobei der spitz zulaufende untere Teil (22) dieses Behälters die entlüftete Probe enthält, und
- einen Sensor (16) für die Konzentration, der den entlüfteten Zentrifugenablauf aus diesem spitz zulaufenden unteren Teil (22) des Behälters (20) aufnimmt,
umfaßt.

5. Vorrichtung nach Anspruch 4, **dadurch gekennzeichnet, daß** der Entlüftungsbehälter (20) mit Mitteln zur Entfernung von Schaum ausgerüstet ist, die in Form eines langsamen Rührers (38) ausgeführt sind.

6. Vorrichtung nach Anspruch 4 oder 5, **dadurch gekennzeichnet, daß** der Sensor (16) - mit einer bekannten Bauart - für die Konzentration in einem zweiten Behälter (24) angebracht ist, der den aus dem Entlüftungsbehälter (20) stammenden Zentrifugenablauf in seinem unteren Teil aufnimmt.

7. Vorrichtung nach einem der Ansprüche 4 bis 6, **dadurch gekennzeichnet, daß** sie ein System für die schnelle Reinigung und Spülung enthält, das entsprechend eines festgelegten Zyklus arbeitet, um die mögliche Bildung und Ansammlung von Schaum auf der Oberfläche im Behälter zu entfernen.

8. Vorrichtung nach Anspruch 7, **dadurch gekennzeichnet, daß** das periodisch arbeitende Schnellreinigungssystem Mittel zur vollständigen und schnellen Entleerung des Entlüftungsbehälters (20) und des den Konzentrationssensor enthaltenden zweiten Behälters (24) und Mittel (34, 36) für das Einleiten von Druckwasser in diese Behälter, um darin für den Spülvorgang zu sorgen, umfaßt, wobei während dieser Reinigungs- und Spülphase sowie während der auf diese Reinigungsphase folgenden Füllphase das Sensorsignal gespeichert wird.

## Claims

1. Process for the continuous measurement of the concentration of suspended materials or the turbidity of a centrifugate coming from a centrifuge or a dehydration machine, according to which a sample of the centrifugate removed by continuous pumping is supplied to a deaeration enclosure, characterized in that said centrifugate sample is supplied tangentially into the upper part of said enclosure (20), the rising speed of gas bubbles separated from the centrifugate in said enclosure is at least twice higher than the speed of the liquid admitted into the enclosure and the measurement of the concentration takes place on a completely deaerated centrifugate, whose composition is identical to that of the sample removed.

2. Process according to claim 1, characterized in that the passage speed of the centrifugate sample in the deaeration enclosure (20) is approximately 0.1 to 0.5 cm/sec.

3. Process according to one of the claims 1 or 2, characterized in that the rising speed of the gas bubble separated from the centrifugate in the deaeration enclosure (20) is approximately 0.6 to 5 cm/sec.

4. Device for performing the process according to any one of the claims 1 to 3, characterized in that it has a cylindroconical deaeration enclosure (20), into the upper part of which is delivered the centrifugate sample, the conical lower area (22) of said enclosure receiving the deaerated sample and a concentration sensor (16) receiving the deaerated centrifugate from the conical, lower area (22) of said enclosure (20).

5. Device according to claim 4, characterized in that said deaeration enclosure (20) is provided with means for eliminating foam in the form of a slow stirrer (38).

6. Device according to either of the claims 4 and 5, characterized in that the known concentration sensor (16) is placed in a second enclosure (24) receiving in its lower part the centrifugate coming from the deaeration enclosure (20).

7. Device according to any one of the claims 4 to 6, characterized in that it has a fast cleaning and rinsing system operating according to a predetermined cycle in order to eliminate the formation and possible accumulation of foam on the surface of said enclosure.

8. Device according to claim 7, characterized in that said fast cleaning system of a periodic nature has means for ensuring a complete, rapid emptying of the deaeration enclosure (20) and the second enclosure (24) containing the concentration sensor, and means (34, 36) for admitting pressurized water into said enclosures so as to ensure the rinsing, the sensor signal being stored during said cleaning and rinsing phase, as well as during the filling stage following onto said cleaning phase.
